# EUROPÄISCHE PATENTSCHRIFT

(11) **EP 0 606 658 B1**
(45) Veröffentlichungstag und Bekanntmachung des Hinweises auf die Patenterteilung: **01.05.1996**
(21) Anmeldenummer: 93121131.2
(22) Anmeldetag: 30.12.1993
(51) Int. Cl.: C07C 311/08, C07D 295/12, A61K 31/18, A61K 31/40, A61K 31/45, A61K 31/495, A61K 31/535, A61K 31/55

(54) **Substituierte N-Aminoalkylmethansulfanilide als Antispasmodika**
Substituted N-aminoalkylmethansulphoamides as antispasmodic agents
N-Aminoalkylméthanesulfonamides substitués comme agents antispasmodiques

(30) Priorität: 13.01.1993 DE 4300696
(43) Veröffentlichungstag der Anmeldung: 20.07.1994
(73) Patentinhaber: ROEMMERS S.A.I.C.F., Buenos Aires 1086 (AR)
(72) Erfinder: Monti, Carlos Ernesto Antonio, Buenos Aires (AR); Aldomá, Gustavo Enrique, 1426 Buenos Aires (AR)
(74) Vertreter: Wibbelmann, Jobst, Dr., Dipl.-Chem.

(56) Entgegenhaltungen:
- EP-A- 0 330 065
- US-A- 3 840 597

## Beschreibung

Die vorliegende Erfindung bezieht sich auf neue substituierte N-Aminoalkylmethansulfanilide, deren Lösungen und pharmazeutisch verträgliche Additionssalze, ihre pharmazeutischen Zusammensetzungen und deren therapeutische Verwendung, insbesondere als Antispasmodika.

### STAND DER TECHNIK

Es sind verschiedene pharmazeutische Wirkgrundsätze bekannt, die in der Therapeutik für die Herstellung von Antispasmodika, das heißt Medikamenten, die Spasmen der glatten Muskulatur lindern, verwendet werden. Diese Wirkstoffe werden in zwei Gruppen unterteilt: Anticholinergika (oder Neurotrope) und Muskulotrope.

Die bekannten antispasmodischen Wirkstoffe vom anticholinergenen Typ, die gegen Acetylcholin in den Muscarinrezeptoren wirken, umfassen natürliche Alkaloide (Atropin, Escopolamin, Hyoscyamin), natürliche Alkaloidderivate (Butylescopolamin-Bromid, Octatropin-Methylbromid), synthetische tertiäre Amine (Amykelin, Trimebutin) und synthetische quaternäre Ammoniumsalze (Clydinbromid, Pinaverium-, Propantelin- und Valetamat-Bromid). Diese Gruppe von antispasmodischen Wirkstoffen weist die klinische Schwierigkeit auf, daß sie verschiedene unerwünschte Nebenwirkungen aufweist wie Mydriasen, verwischtes Sehvermögen, Konstipation, Inhibition der Speichelsekretion und Tachykardie.

Die bekannten antispasmodischen Agenzien vom muskulotropen Typ, die durch einen überhaupt nicht bekannten Mechanismus auf den Muskel wirken, enthalten Mebeverin, Papaverin und Pramiverin. Der klinische Nutzen dieser Pharmakagruppe wurde in Frage gestellt, und ihre Verwendung wird im allgemeinen Fällen vorbehalten, in denen Anticholinergika kontraindiziert sind. Rociverin war das erste Antispasmodikum, bei dem über ein Gleichgewicht zwischen der anticholinergenen und der muskulotropen Wirkung berichtet wurde.

Somit ist das Problem, neue antispasmodische Produkte mit geringen unerwünschten Nebenwirkungen zu finden, in der Therapie von Mensch und Tier nicht vollständig gelöst.

Die vorliegende Erfindung löst dieses Problem, indem sie eine Gruppe von chemisch neuen Produkten vom Typ substituierter N-Aminoalkylmethansulfanilide zur Verfügung stellt, die insbesondere in der Therapie als antispasmodische Wirkstoffe eingesetzt werden. Es gibt keine strukturelle Ähnlichkeit zwischen den Produkten der vorliegenden Erfindung und den im Stand der Technik bekannten Antispasmodika. Die vom chemischen Standpunkt her anscheinend nächstliegenden Produkte sind im US-Patent 3.840.597 beschrieben. Keines dieser Produkte enthält jedoch eine N-Aminoalkyl-Gruppe in seiner Struktur, und andererseits werden diese lediglich als entzündungshemmende Wirkstoffe verwendet, ohne daß jemals die Möglichkeit ihrer Verwendung als Spasmolytika beschrieben wurde.

### BESCHREIBUNG

Der Gegenstand der vorliegenden Erfindung besteht darin, ein substituiertes N-Aminoalkylmethansulfanilid gemäß Formel (I), eine Lösung oder ein Salz zur Verfügung zu stellen, deren Zugabe pharmazeutisch akzeptabel ist,

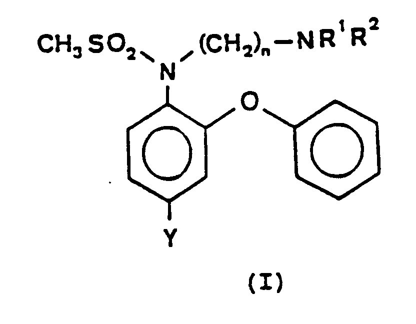

### Hierbei entspricht

Y Wasserstoff, Cyano, Nitro, Amino, Acetamid oder Halogen;
n ist eine ganze Zahl zwischen 2 und 4;
R¹ und R², gleich oder verschieden, sind (C1-C4)-Alkyl oder sie werden miteinander und mit dem N-Atom verbunden, um vorzugsweise einen 1-Pyrrolidinyl, 1-Piperidinyl, 1-Piperazinyl, 4-Methyl-1-Piperazinyl, 4-Ethylpiperazinyl, 4-Propylpiperazinyl, 1-Homopiperidinyl (1-Azacycloheptanyl) oder 4-Morpholin-Rest zu bilden.

Gemäß der vorliegenden Erfindung werden vorzugsweise die Produkte der Formel (I) verwendet, wobei n 2 oder 3 ist, sowie diejenigen, bei denen Y Nitro, Cyano oder Halogen ist. Vorzuziehen sind insbesondere diejenigen, bei denen R¹ und R², die gleich oder verschieden sind, Methyl, Ethyl oder Isopropyl sind, oder miteinander und mit dem N-Atom verbunden werden, um einen 1-Pyrrolidinyl, 1-Piperidinyl, 1-Homopiperidinyl (1-Azacycloheptanyl) oder 4-Morpholin-Rest zu bilden, wobei 1-Piperidinyl der bevorzugteste Rest ist.

Die bevorzugtesten Produkte der vorliegenden Erfindung sind diejenigen, die der allgemeinen Formel (I) entsprechen und ausdrücklich in den Beispielen (Tabelle 1) angegeben sind. Diese Produkte werden hier zum ersten Mal beschrieben.

Die Produkte (I) der vorliegenden Erfindung dienen der Therapie, insbesondere als Antispasmodika, weshalb die Verwendung dieser Produkte für die Herstellung von antispasmolytischen Medikamenten ebenfalls Gegenstand der vorliegenden Erfindung ist.

Die Verabreichung der Produkte (I) der vorliegenden Erfindung an Menschen oder Tiere erfolgt in Form von Arzneimittelzusammensetzungen, die therapeutisch wirksame Mengen des Produktes und geeignete Mengen von pharmazeutisch verträglichen Hilfsstoffen enthalten. Gegenstand der vorliegenden Erfindung ist ebenfalls ein Verfahren zur Zubereitung von Arzneimittelzusammensetzungen, wozu auch das Mischen von therapeutisch wirksamen Mengen von Produkten (I) mit den gewünschten Mengen geeigneter Hilfsstoffe gehört.

Gegenstand der vorliegenden Erfindung ist es ebenfalls, ein Verfahren für die Zubereitung eines Produktes (I) zu liefern, das Folgendes umfaßt:
A) Wenn Y Wasserstoff oder Nitro ist, muß - jeweils - Sulfanilid (II) oder (III) mit T-(CH₂)ₙ-NR¹R² reagieren, wobei T eine Austrittsgruppe ist, die vom N der Sulfanilide (II) oder (III) verdrängt werden kann; 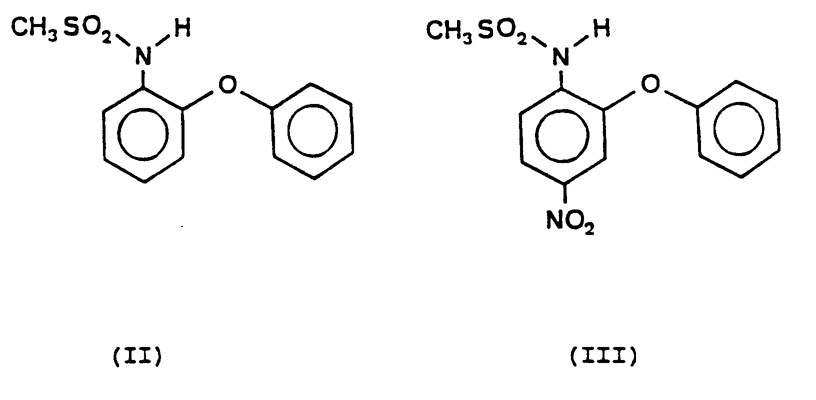
B) Wenn Y Amino ist, wird mittels katalytischer Hydrierung das entsprechende Produkt (I) reduziert, bei dem Y Nitro entspricht;
C) Wenn Y Acetamid ist, reagiert das entsprechende Produkt (I), bei dem Y Amino ist, mit einem T-COCH₃-Reagens, bei dem T eine Austritts-Gruppe ist;
D) Wenn Y Cyano oder Halogen ist, wird das entsprechende Produkt (I), bei dem Y Amino ist, einer Sandmeyerreaktion (HN0₂ und Kupfer-Zyanid oder Kupfer-Halogenid) unterzogen; und wahlweise wird die notwendige Säure zur Bildung des gewünschten Additionssalzes hinzugegeben.

Bei der Methode A ist die Austritts-Gruppe T vorzugsweise Chlor, Brom, Methylsulphonyloxi oder 4-Methylphenylsulphonyloxi. Die Reaktion erfolgt am besten bei Vorhandensein einer equimolaren oder überschüssigen Menge einer anorganischen Base, vorzugsweise eines Alkali- oder Erdalkali-Metalles wie Natriumcarbonat, Kaliumcarbonat, Bariumcarbonat, Wasserstoffkaliumcarbonat oder Natriumhydroxid; oder auch in Anwesenheit einer tertiären organischen Base wie Triethylamin oder Pyridin. Als Lösungsmittel eignet sich vorzugsweise ein niederer Alkohol wie Ethanol, Isopropanol (vorzuziehen) oder sekundärer Butylalkohol; ein Keton wie Aceton (vorzuziehen), Methylethylketon, Methylisobutylketon oder Cyclohexanon; oder ein dipolares Lösungsmittel wie DMF, DMS0 oder Acetonitril. Die Temperaturen liegen vorzugsweise zwischen Raumtemperatur und 150°C, und die Reaktionszeiten vorzugsweise zwischen 1 h und mehreren Tagen.

Bei der Methode B erfolgt die katalytische Hydrierung vorzugweise bei Drücken zwischen 1 und 5 atm; bevorzugt wird insbesondere der Druck von circa 2 atm; der Katalysator kann Palladium (vorzuziehen), Platin oder Platinoxid sein. Vorzugsweise dauert die Reaktion zwischen 30 Min. und mehreren Stunden, und sie erfolgt bei Temperaturen zwischen Raumtemperatur und 60 °C. Die Lösungsmittel sind vorzugsweise niedere Alkohole, insbesondere Ethanol Wasser oder Essigsäure.

Bei der Methode C ist das T des Reagenzes T-COCH₃ vorzugsweise Chlor oder OCOCH₃. Die Reaktion erfolgt in Anwesenheit einer Base, die, wenn sie flüssig ist, als Lösungsmittel wirken kann. Andere bevorzugte Lösungsmittel sind aromatische Kohlenwaserstoffe wie Toluen oder Xylen, Ether, wie Dioxan, halogenierte Kohlenwasserstoffe wie Dichlormethan oder Chloroform. Die Reaktion erfolgt vorzugsweise bei Temperaturen zwischen -20 und 150 °C über einen Zeitraum von 1 bis 24 h.

Bei der Methode D laufen die typischen Bedingungen der Sandmeyer-Reaktion ab.

Die Produkte (I) können wie freie Basen oder vorzugsweise als pharmazeutisch vertragliche Additionssalze von organischen oder anorganischen Säuren isoliert oder gereinigt werden. Bevorzugt werden die Salze der Salz- oder Oxalsäure.

Die nachstehenden Ausführungsbeispiele sollen die Erfindung erläutern:
Die Versuchsbeispiele für die antispasmodische Wirkung geben an, daß die eingesetzten Produkte (I) einen therapeutischen Nutzen bringen.

### BEISPIELE

### Beispiele für die Synthesemethode A: Herstellung des Hydrochlorids von N-2-(Dimethylamino)-Ethyl-2-Phenoxy-4-Nitromethansulfanilid (1)

Eine Mischung von 3,08 g 2-Phenoxy-4-Nitromethansulfanilid (beschrieben in US 3.840.597) wurde mit 4,24 g Natriumcarbonat in 80 ml Aceton im Rückfluß über 6 Stunden zur Reaktion gebracht. Dann wurden 2,88 g von 2-Chlorethyldimethylamin-Hydrochlorid hinzugegeben, und es wurde über 30 h bei kräftigem Rühren ein Rückfluß aufrechtgehalten. Danach wurde gefiltert und das Lösungsmittel verdampft. Der Rückstand wurde in Dichlormethan gelöst und mehrmals mit einer mit NaCL gesättigten wässrigen Lösung gewaschen. Das Lösungsmittel der organischen Phase wurde verdampft. Der Rückstand wurde in Isopropanol gelöst. Der somit erhaltenen Lösung wurde HCl bis zu einem pH von circa 2,0 beigemengt, wobei man die obige Verbindung mit einer Ausbeute von 89 % erhält. Nach einer Rekristallisation in Isopropanol wurde ein Schmelzpunkt von 128-30 °C ermittelt.

Die Produkte 2 - 8 und 24 in Tabelle 1 wurden in analoger Weise erhalten, d. h. ausgehend von 2-Phenoxy-4-Nitromethansulfanilid und den entsprechenden Choralkylamin-Hydrochloriden mit der Formel Cl-(CH₂)ₙ-NR¹R². In der genannten Tabelle sind die erzielte Ausbeute und die festgestellten Schmelzpunkte angegeben.

### Herstellung von N-[2-(4-Morpholinyl)Ethyl]-2-Phenoxymethansulfanilid-Hydrochlorid (13)

Eine Mischung von 1,32 g 2-Phenoxymethansulfanilid (beschrieben in US 3.840.597) und 2,12 g Natriumcarbonat in Aceton wurde 6 h im Rückfluß gehalten.Es wurden 1,86 g N-(2-Chlorethyl)-Morpholin-Hydrochlorid beigemengt und 25 Stunden im Rückfluß gehalten. Danach wird das Reaktionsgemisch auf Raumtemperatur abgekühlt, gefiltert und das Lösungsmittel verdampft. Der Rückstand wurde in Dichlormethan gelöst, mehrmals mit Wasser gewaschen und das Lösungsmittel der organischen Phase verdampft. Der Rückstand wurde in absolutem Ethanol gelöst und mit HCl angesäuert (10 % in Ethanol), wobei das obige Produkt (13) mit einer Ausbeute von 91 % erhalten wurde. Nach Rekristallisation wurde ein Schmelzpunkt von 173-4 °C festgestellt.

Die Produkte 14-18 in Tabelle 1 wurden auf analoge Weise erhalten, d. h. ausgehend von 2-Phenoxy-Methansulfanilid und den jeweiligen Chloralkylamin-Hydrochloriden nach der Formel Cl-(CH₂)ₙ-NR¹R². In der genannten Tabelle sind die erhaltenen Ausbeuten und die ermittelten Schmelzpunkte angegeben.

**Tabelle 1**

| Hergestellte Produkte 1 - 21: Hydrochloride nach Formel (I) | | | | | |
|---|---|---|---|---|---|
| Nr. | n. | Y | NR¹R² | Ausbeute (%) | Schmelzpunkt (°C) |
| 1 | 2 | NO₂ | N(CH₃)₂ | 89 | 128-30 |
| 2 | 2 | NO₂ | N(CH₂CH₃)₂ | 83 | 155-7 |
| 3 | 2 | NO₂ | 1-Piperidinyl | 88 | 213-5 |
| 4 | 2 | NO₂ | 1-Pyrrolidinyl | 81 | 157-9 |
| 5 | 2 | NO₂ | N(CH(CH₃)₂)₂ | 68 | 195-7 |
| 6 | 2 | NO₂ | 4-Morpholinyl | 89 | 245-7 |
| 7 | 2 | NO₂ | 4-Methyl-1-Piperazinyl | 39 | 246-8 |
| 8 | 3 | NO₂ | 1-Piperidinyl | 75 | 204-8 |
| 9 | 2 | NH₂ | 1-Piperidinyl | 90 | 220-2 |
| 10 | 2 | NH₂ | N(CH₂CH₃)₂ | 80 | 160-2 |
| 11 | 2 | NHCOMe | N(CH₂CH₃)₂ | 87 | 183-5 |
| 12 | 2 | NHCOMe | 1-Piperidinyl | 98 | 158-9 |
| 13 | 2 | H | 4-Morpholinyl | 91 | 173-4 |
| 14 | 2 | H | N(CH₃)₂ | 67 | 196-8 |
| 15 | 2 | H | N(CH₂CH₃)₂ | 78 | 180-2 |
| 16 | 2 | H | 1-Piperidinyl | 50 | 152-4 |
| 17 | 2 | H | N CH(CH₃)₂)₂ | 47 | 165-7 |
| 18 | 2 | H | 1-Pyrrolidinyl | 51 | 165-7 |
| 19 | 2 | CN | 1-Piperidinyl (Oxalat) | 65 | 206-8 |
| 20 | 3 | NH₂ | 1-Piperidinyl | 91 | 142-4 |
| 21 | 3 | CN | 1-Piperidinyl | 27 | 208-10 |
| 22 | 3 | I | 1-Piperidinyl | 55 | 176-8 |
| 23 | 3 | I | 1-Piperidinyl | 25 | 80-2 |
| 24 | 4 | NO₂ | 1-Piperidinyl | 92 | 100-3 |
| 25 | 4 | NH₂ | 1-Piperidinyl | 85 | 216-8 |
| 26 | 2 | Br | 1-Piperidinyl | 64 | 174-5 |
| 27 | 3 | Br | 1-Piperidinyl | 33 | 154-6 |
| 28 | 4 | Br | 1-Piperidinyl | 32 | 155-7 |
| 29 | 2 | NO₂ | 1-Homopiperidinyl | 71 | 169-70 |

### Beispiele für die Synthesemethode B: Herstellung von N-[2-(1-Piperidinyl)Ethyl]-4-Amino-2-Phenoxymethansulfanilid-Hydrochlorid (9)

Ein Hydrierungskolben wurde mit 4,56 g N-[2-(1-Piperidinyl)Ethyl]-4-Nitro-2-Phenoxymethansulfanilid-Hydrochlorid (3) gefüllt zusammen mit 0,5 g Pd (5 % in C) und 300 ml 0.2 HCl. In einer H₂-Atmosphäre von mit 3.42 x 10⁵ Bar (50 psi) und unter Rühren erfolgte die Reaktion 3 h lang bei Raumtemperatur. Danach wurde durch ein Celitebett gefiltert und das Lösungsmittel des Filtrates verdampft und das obige Produkt mit einer Ausbeute von 90 % erhalten. Nach Rekristallisation in absolutem Ethanol wurde der Schmelzpunkt mit 220-2 °C bestimmt.

Analog dazu und ausgehend von N-[2-(Diethylamino)Ethyl]-4-Nitro-2-Phenoxymethansulfanilidin-Hydrochlorid (2) erhielt man N-(2-(Diethylamino)Ethyl)-4-Amino-2-Phenoxymethansulfanilidin-Hydrochlorid (10) mit einer Ausbeute von 80 % und einem Schmelzpunkt von 160-2 °C.

Analog dazu und ausgehend von N-[3-(1-Piperidinyl)Propyl]-4-Nitro-2-Phenoxymethansulfanilid-Hydrochlorid (8) erhielt man N-[2-(1-Piperidinyl)Propyl]-4-Amino-2-Phenoxymethansulfanilid-Hydrochlorid (20) mit einer Ausbeute von 91 % und einem Schmelzpunkt von 142-4.

### Beispiele für die Synthesemethode C: Herstellung des N-[2-(Diethylamino)Ethyl]-4-Acetamid-2-Phenoxymethansulfanilid-Hydrochlorids (11)

Eine Lösung von 1,8 g N-[2-(Diethylamino)Ethyl]-4-Amino-2-Phenoxymethansulfanilid-Hydrochlorid (10) wurde in 20 ml Wasser mit 0,6 g Essigsäureanhydrid bei 25 °C 3 h lang zum Reagieren gebracht. Die Reaktionsmischung wurde mit Natriumhydroxid alkalisch gemacht und mit Dichlormethan extrahiert. Der organische Extrakt wurde mehrmals mit Wasser gewaschen, mit Natriumsulfat getrocknet und das Lösungsmittel verdampft. Der Rückstand wurde in absolutem Ethanol gelöst und eine Lösung von HCl (10 % in Ethanol) zugegeben, womit das obengenannte Produkt (1) mit einer Ausbeute von 87 % erhalten wurde. Nach Rekristallisation wurde der Schmelzpunkt mit 183-5 °C bestimmt.

Analog dazu und ausgehend von N-[2-(1-Piperidinyl)Ethyl]-4-Amino-2-Phenoxymethansulfanilid-Hydrochlorid (9) wurde ein N-[2-(1-Piperidinyl)Ethyl]-4-Acetamido-2-Phenoxymethansulfanilid-Hydrochlorid (12) mit einer Ausbeute von 98 % und einem Schmelzpunkt von 158-9 °C erhalten.

### Beispiele für die Synthesemethode D (Sandmeyer-Reaktion: Herstellung von N-[2-(1-Piperidinyl)Ethyl]-4-Cyano-2-Phenoxymethansulfanilid-Oxalat (19)

Zu einer Mischung von o,42 g N-[2-(1-Piperidinyl)Ethyl]-4-Amino-2-Phenoxymethansulfanilid-Hydrochlorid (9), 1 ml HCl (conc.) und 5 ml Wasser bei 0-5 °C wurde eine Lösung von 0,42 g Natriumnitrit in 10 ml Wasser getropft, das Ganze wurde nach der Zugabe 20 Min. gerührt. Die Mischung wurde mit NaHC0₃ neutralisiert, und zu der Lösung wurde langsam eine Lösung von 2,6 g Kaliumzyanid und 1 g Kupferchlorid in 10 ml Wasser bei 5-10 °C zugegeben. Die Mischung wurde 30 Min. bei Raumtemperatur und gehalten und dann 30 Min. auf 50 °C erwärmt. Das auf Raumtemperatur abgekühlte Reaktionsgemisch wurde mit Ethylacetat extrahiert. Die organische Phase wurde mit Wasser gewschen, getrocknet und das Lösungsmittel verdampft. Der Rückstand wurde in 10 m Ethanol gelöst, und der Lösung wurde 0,6 g wasserfreie Oxalsäure beigemengt. Sie wurdte 1 h bei Raumtemperatur gehalten und danach 2 h in einem Eisbad. Sie wurde gefiltert und mit kaltem Ethanol gewaschen, wobei das obengenannte Produkt (19) mit einer Ausbeute von 65 % erlangt wurde. Nach Rekristallisation wurde der Schmelzpunkt mit 205-8 °C bestimmt.

Analog dazu und ausgehend von N-[3-(1-Piperidinyl)Propyl]-4-Amino-2-Phenoxymethansulfanilid-Hydrochlorid (20) unter Beimengung von HCl anstelle der Oxalsäure erhielt man ein N-[3-(1-Piperidinyl)Propyl]-4-Cyano-2-Phenoxymethansulfanilid-Hydrochlorid (21) mit einer Ausbeute von 27 % und einem Schmelzpunkt von 208-10 °C. Ausgehend von Amino (20), wobei jedoch das Kupfercyanid durch Kupferjodid ersetzt wurde, erhielt man das Produkt (23); und ausgehend von Amino (9) erhielt man das Produkt (22) mit der Ausbeute und den Schmelzpunkten, die in der Tabelle angegeben sind.

### Anwendungsbeispiele: Versuche zur antispasmodischen Wirkung

Mit jedem der in Tabelle 2 genannten Produkte wurden Versuche zur antispasmodischen Wirkung durchgeführt, einer im Vergleich zu Acetylcholin (Ac-Col) und ein weiterer gegenüber BaCl₂ (cf. G. Toson et al., Arzneim. Forsch. 1978, Band 28, (II), Seite 1130).

Der Versuch zur inhibitorischen Wirkung bei der durch Acetylcholin (Ac-Col) induzierten Kontraktion erfolgte beim Ileus der männlichen Winstar-Ratte mit einem Gewicht von 200-300 g in einem 10 ml Bad mit einer Tyrode-Lösung bei 37 °C, gasgespült mit 95 % 0₂ und 5 % N₂ und einem Gramm Spannung. Die Kontraktionsreaktion wurde mit einem isotonischen Wandler gemessen. Die Kontraktion wurde mit Acetylcholin in zunehmenden Dosen von 10⁻⁹ bis 10 ⁻² M induziert. Die Versuchsprodukte wurden 5 Min. vor Acetylcholin gegeben. Es wurden drei oder mehrere Dosispegel angewendet, und die DE₅₀ wurden gemessen und in Molkonzentrationen (M) angegeben.

Der Versuch zur Inhibitionswirkung bei der durch BaCl₂ induzierten Kontraktion erfolgte unter analogen Bedingungen, wobei die Kontraktion jedoch in zunehmenden Dosen zwischen 5 x 10⁻⁵ bis 10⁻³ M ausgelöst wurde.

**Tabelle 2**

| Antispasmodische Wirkung einiger Producte in vitro | | |
|---|---|---|
| Nr. | DE50 (10⁻⁶ M) vs. Ac-Col. | DE50 (10⁻⁶ M) vs BaCl₂ |
| 1 | 4.99 | 6.45 |
| 2 | 12.4 | 9.37 |
| 3 | 9.38 | 3.65 |
| 4 | 18.4 | 7.31 |
| 5 | 4.89 | 5.06 |
| 6 | 2.5 | 134 |
| 8 | 9.08 | 8.64 |
| 15 | 13.9 | 11.5 |
| 16 | 26.9 | 10.9 |
| 17 | 11.2 | 7.37 |
| 19 | 9.22 | 9.79 |
| 21 | 8.94 | 14.4 |
| 23 | 0.90 | 1.04 |
| 26 | 1.00 | 0.94 |
| 27 | 1.30 | 1.90 |
| 28 | 5.65 | 4.78 |
| 29 | 1.58 | 1.61 |

## Patentansprüche

1. Verbindung der Formel (I), deren Lösungen und phamazeutisch verträgliche Additionssalze 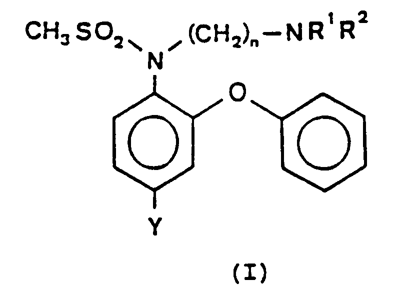 wobei
Y Wasserstoff, Cyano, Nitro, Amino, Acetamid oder Halogen ist;
n eine ganze Zahl zwischen 2 und 4 ist;
R¹ und R², die gleich oder verschieden sind, (C1-C4)-Alkyl sind, oder miteinander oder über das N-Atom verbunden sind, um vorzugsweise einen 1-Pyrrolidinyl, 1-Piperidinyl, 1-Piperazinyl, 4-Methyl-1-Piperazinyl, 4-Ethylpiperazinyl, 4-Propylpiperazinyl, 1-Homopiperidinyl oder 4-Morpholin-Rest zu bilden.

2. Eine Verbindung gemäß Anspruch 1, deren Lösung oder pharmazeutisch verträgliches Additionssalz, wobei n 2 oder 3 ist.

3. Eine Verbindung gemäß Anspruch 2, deren Lösung oder pharmazeutisch verträgliches Additionssalz, wobei R¹ und R², die gleich oder verschieden sind, Methyl, Ethyl oder Isopropyl sind, oder miteinander oder über das N- Atom verbunden sind, um einen 1-Pyrrolidinyl, 1-Piperidinyl, 1-Homopiperidinyl oder 4-Morpholin-Rest zu bilden.

4. Eine Verbindung gemäß einem der Ansprüche 1-3, deren Lösung oder pharmazeutisch verträgliches Additionssalz, wobei Y Nitro, Cyano oder Halogen ist.

5. Eine Verbindung gemäß einem der Ansprüche 1-4, deren Lösung oder pharmazeutisch verträgliches Additionssalz, wobei NR¹R² 1-Piperidinyl ist.

6. Hydrochlorid von einer der Verbindungen gemäß den Ansprüchen 1-5.

7. Produkt gemäß den Ansprüchen 1-6 zur therapeutischen Anwendung.

8. Produkt gemäß Anspruch 7 für die Anwendung als spasmolytisches Agens.

9. Pharmazeutische Zusammensetzung, die eine therapeutisch wirksame Menge eines Produktes gemäß einem der Ansprüche 1-6 und geeignete Mengen an pharmazeutisch akzeptablen Hilfsstoffen enthält.

10. Verwendung eines Produktes gemäß einem der Ansprüche 1-6 für die Herstellung eines Antispasmodikums.

11. Verfahren zur Herstellung einer Verbindung (I) gemäß Anspruch 1, das Folgendes umfaßt:
A) wenn Y Wasserstoff oder Nitro ist, wird - jeweils - Sulfanilid (II) oder (III) mit T-(CH₂)ₙ-NR¹R²) zum Reagieren gebracht, wobei T einer Austritts-Gruppe entspricht, die vom N der Sulfanilide (II) oder (III) verdrängt werden kann; 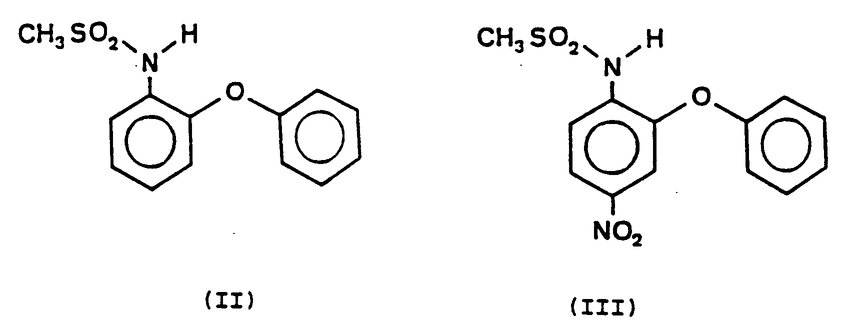
B) wenn Y Amino ist, erfolgt die Reduktion des entsprechenden Produktes (I) mittels katalytischer Hydrierung, wobei Y Nitro entspricht;
C) Wenn Y Acetamid ist, wird das jeweilige Produkt (I), bei dem Y Amino entspricht, mit einem T-COCH₃-Reagens zum reagieren gebracht, wobei T eine Austritts-Gruppe ist;
D) Wenn Y Cyano oder Halogen ist, wird das entsprechende Produkt (I), bei dem Y Amino ist, einer Sandmeyer-Reaktion unterworfen (HN0₂ und Kupfercyanid oder Kupfer-Halogenid);
und wahlweise wird die zur Bildung des gewünschten Additionssalzes notwendige Säure hinzugefügt.

12. Verfahren zur Herstellung einer Zusammensetzung gemäß Anspruch 9, das das Mischen einer therapeutisch wirksamen Menge eines Produktes gemäß einem der Ansprüche 1-6 mit den gewünschten Mengen von geeigneten Excipientien umfaßt.

## Claims

1. Compound of formula (I), solution and pharmaceutically acceptable addition salts thereof 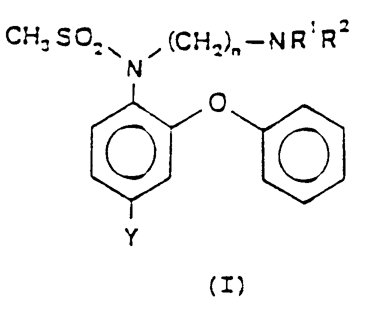 wherein
Y represents hydrogen, cyano, nitro, amino, acetamide or halogen;
n is an integer between 2 and 4;
R¹ and R², which are equal or different are (C1-C4)-alkyl or are connected with each other and via the N-atom, thereby forming preferably a 1-pyrrolidinyl, 1-piperidinyl, 1-piperazinyl, 4-methyl-1-piperazinyl, 4-ethylpiperazinyl, 4-propylpiperazinyl, 1-homopiperidinyl or 4-morpholine residue.

2. Compound according to claim 1, solution or pharmaceutically acceptable additon salt thereof, wherein n is 2 or 3.

3. Compound according to claim 2, solution or pharmaceutically acceptable addition salt thereof, wherein R¹ and R² which are equal or different are methyl, ethyl or isopropyl or are connected with each other and via the N-atom, thereby forming a 1-pyrrolidinyl, 1-piperidinyl, 1-homopiperidinyl or 4-morpholine residue.

4. Compound according to any one of claims 1 to 3, solution or pharmaceutically acceptable addition salt thereof, wherein Y represents nitro, cyano or halogen.

5. Compound according to any one of claims 1 to 4, solution or pharmaceutically acceptable addition salt thereof, wherein NR¹R² represents 1-piperidinyl.

6. Hydrochloride of one of the compounds according to any one of claims 1 to 5.

7. Product according to any one of claims 1 to 6 for therapeutical application.

8. Product according to claim 7 for the application as spasmolytic agent.

9. Pharmaceutical composition containing a therapeutically effective amount of a product according to any one of claims 1 - 6 and a suitable amount of a pharmaceutically acceptable excipient.

10. Method of treatment with a therapeutically effective amount of an antispasmodic agent wherein a product according to any one of claims 1 - 6 is administered.

11. Process for the preparation of a compound (I) according to claim 1, comprising the following:
A) when Y is hydrogen or nitro, sulfanilide (II) or (III), respectively, is reacted with T-(CH₂)ₙ-NR¹R², wherein T represents a leaving group that can be displaced from N of sulfanilides (II) or (III); 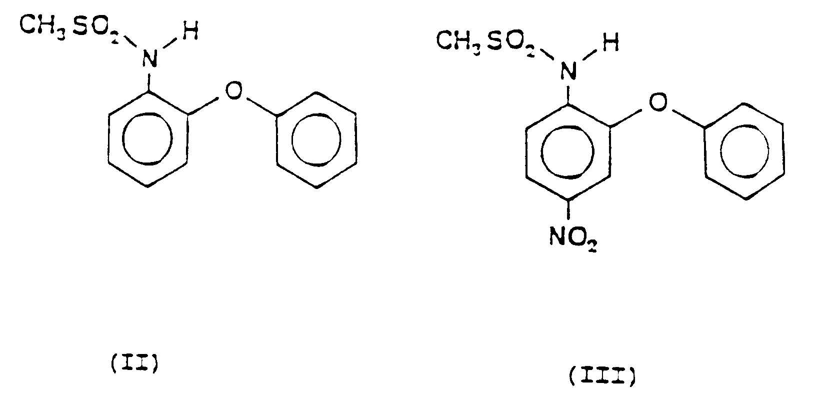
B) when Y represents amino, the reduction of the corresponding product (I) wherein Y represents nitro is carried out by means of catalytic hydrogenation;
C) when Y represents acetamide, the corresponding product (I) wherein Y represents amino is reacted with a T-COCH₃-reagent, wherein T is a leaving group;
D) when Y represents cyano or halogen, the corresponding product (I) wherein Y represents amino is subjected to a Sandmeyer reaction (HNO₂ and copper cyanide or copper halogenide);
and wherein optionally the amount of acid necessary for the formation of the desired addition salt is added.

12. Process for the preparation of a composition according to claim 9 which comprises mixing a therapeutically effective amount of a product according to any one of claims 1 - 6 with the desired amount of a suitable excipient.

## Revendications

1. Composé de formule (I), ses solutions et ses sels d'addition pharmaceutiquement acceptables 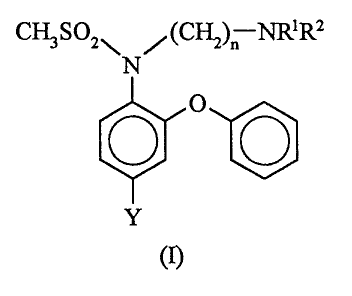 où
Y représente un atome d'hydrogène, un groupe cyano, nitro, amino ou acétamide ou un atome d'halogène;
n est un nombre entier compris entre 2 et 4;
R¹ et R², qui sont identiques ou différents, représentent des groupes alkyle en C₁-C₄, ou sont reliés entre eux ou par l'intermédiaire de l'atome d'azote pour former de préférence un reste 1-pyrrolidinyle, 1-pipéridinyle, 1-pipérazinyle, 4-méthyl-1-pipérazinyle, 4-éthylpipérazinyle, 4-propylpipérazinyle, 1-homopipéridinyle ou 4-morpholinyle.

2. Composé selon la revendication 1, ses solutions ou ses sels d'addition pharmaceutiquement acceptables, dans lesquels n est 2 ou 3.

3. Composé selon la revendication 2, ses solutions ou ses sels d'addition pharmaceutiquement acceptables, dans lesquels R¹ et R², qui sont identiques ou différents, sont des groupes méthyle, éthyle ou isopropyle, ou sont reliés entre eux ou par l'intermédiaire de l'atome d'azote pour former un reste 1-pyrrolidinyle, 1-pipéridinyle, 1-homopipéridinyle ou 4-morpholinyle.

4. Composé selon l'une des revendications 1 à 3, ses solutions ou ses sels d'addition pharmaceutiquement acceptables, dans lesquels Y est un groupe nitro ou cyano ou un atome d'halogène.

5. Composé selon l'une des revendications 1 à 4, ses solutions ou ses sels d'addition pharmaceutiquement acceptables, dans lesquels NR¹R² est un reste 1-pipéridinyle.

6. Chlorhydrate d'un des composés selon les revendications 1 à 5.

7. Produit selon les revendications 1 à 6 pour l'utilisation en thérapeutique.

8. Produit selon la revendication 7 pour l'utilisation comme agent antispasmodique.

9. Composition pharmaceutique qui contient une quantité thérapeutiquement efficace d'un produit selon l'une des revendications 1 à 6 et des quantités appropriées d'agents auxiliaires pharmaceutiquement acceptables.

10. Utilisation d'un produit selon l'une des revendications 1 à 6 pour la préparation d'un antispasmodique.

11. Procédé de préparation d'un composé (I) selon la revendication 1, qui comprend ce qui suit:
A) Lorsque Y est un atome d'hydrogène ou un groupe nitro, on fait réagir respectivement le sulfanilide (II) ou (III) avec T-(CH₂)ₙ-NR¹R², T étant un groupe partant qui peut être déplacé par l'atome N des sulfanilides (II) ou (III): 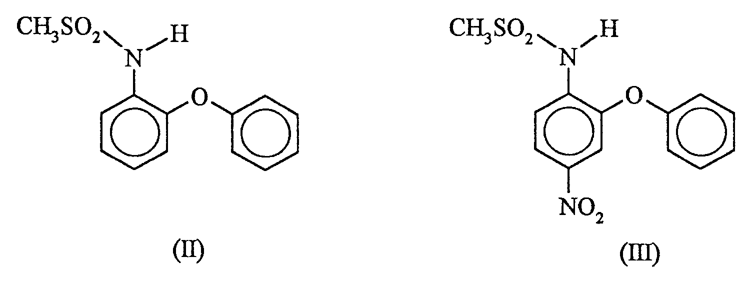
B) Lorsque Y est un groupe amino, on réduit par hydrogénation catalytique le produit (I) correspondant dans lequel Y est un groupe nitro;
C) lorsque Y est un groupe acétamide, on fait réagir le produit (I) correspondant dans lequel Y est un groupe amino avec un réactif T-COCH₃, dans lequel T est un groupe partant;
D) Lorsque Y est un groupe cyano ou un atome d'hydrogène, on soumet le produit (I) correspondant dans lequel Y est un groupe amino à une réaction de Sandmeyer (HNO₂ et du cyanure de cuivre ou un halogénure de cuivre);
et, si on le souhaite, on ajoute l'acide nécessaire pour la formation du sel d'addition d'acide désiré.

12. Procédé de préparation d'une composition selon la revendication 9, qui comprend le mélange d'un quantité thérapeutiquement efficace d'un produit selon l'une des revendications 1 à 6 avec la quantité désirée d'excipients appropriés.
